Europäisches Patentamt

European Patent Office

Office européen des brevets

(11) Publication number: **0 173 478 B1**

## EUROPEAN PATENT SPECIFICATION

(45) Date of publication of patent specification: **02.02.94**

(51) Int. Cl.⁵: **A61K 35/78**, A61K 31/57,
//(A61K35/78,31:57),
(A61K31/57,31:23,31:20)

(21) Application number: **85305552.3**

(22) Date of filing: **05.08.85**

(54) **Treatment of skin disorders.**

(30) Priority: **15.08.84 GB 8420771**

(43) Date of publication of application:
**05.03.86 Bulletin 86/10**

(45) Publication of the grant of the patent:
**02.02.94 Bulletin 94/05**

(84) Designated Contracting States:
**BE CH DE FR GB IT LI LU NL SE**

(56) References cited:
**EP-A- 0 003 407
EP-A- 0 085 579
GB-A- 1 082 624
US-A- 4 305 936**

**Linola-H Fett ( In Klinik und Praxis bewährte
VINCES Preparate, 1905-1959, Ausgabe Mai
1959) Chem.-pharm.-Fabrik**

**Dictionnaire français de Médecine et de
Biologie, A. Manuila et al. Ed. Masson (1981)
p. 38**

**U.M.T. Hootsmuller- Columbinic acid- p. 889**

(73) Proprietor: **SCOTIA HOLDINGS PLC
Efamol House
Woodbridge Meadows
Guildford Surrey GU1 1BA(GB)**

(72) Inventor: **Horrobin, David Frederick
Efamol Limited
Efamol House
Woodbridge Meadows
Guildford Surrey GU1 1BA(GB)**

(74) Representative: **Miller, Joseph et al
J. MILLER & CO.
34 Bedford Row,
Holborn
London WC1R 4JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

FIELD OF INVENTION

The invention relates to problems in anti-inflammatory glucocorticoid treatment of the skin.

BACKGROUND AND EXPLANATION OF INVENTION

Much interest has been shown in recent years in essential fatty acid metabolism, especially in its relation to prostaglandin (PG) metabolism and in particular to the balance of 1-series and 2-series PGs in the body.

The main dietary essential fatty acid (EFA) utilised in the fully healthy human body is linoleic acid, but the delta-6-desaturase that converts it to the next acid in the n-6 series, namely gamma-linolenic acid (GLA) is at a low level of activity in many conditions. The giving of gamma-linolenic acid is beneficial in such conditions and has been the subject of earlier patent applications of the present inventor which discuss the essential fatty acids and their relation to PG metabolism.

Specifically, prostaglandin precursors include linoleic acid, gamma-linolenic acid and dihomo-gamma-linolenic acid (DGLA), conversion in the body being as follows:-

Linoleic acid

$\Delta^6$-desaturase

$\gamma$-Linolenic acid

elongation

Bound reserves ⇌ Dihomo-$\gamma$-linolenic acid

$\Delta^5$-desaturase → Prostaglandins of the 1 series

Bound reserves ⇌ Arachidonic acid (5,8,11,14-eicosatatraenoic acid

→ Prostaglandins of the 2 series

Elongation and desaturation to docosatetraenoic and docosapentaenoic acids of the n-6 series

SKIN CONDITIONS

Many inflammatory skin conditions are associated with excess formation of prostaglandins and cyclo-oxygenase and lipoxygenase products from arachidonic acid. It is generally believed that glucocorticoid ointments and the like are successful in these conditions because (apparently by inhibition of phospholipases concerned) they block the release of arachidonic acid from phospholipid and other bound precursors or reserves. They have a similar effect on DGLA release. Thus glucocorticoid treatment leads to a situation in which both DGLA and arachidonic acid fail to give rise to their metabolites because they are not released from store.

This effect of blocking EFA release from stores the inventor believes desirable with regard to arachidonic acid (of which bodily reserves are large) and probably to explain the therapeutic effects. On the other hand, both free EFAs and PGs and other EFA metabolites play important roles in the skin and their absence is believed to explain some of the undesirable side effects of long term topical steroid treatment

2

such as skin atrophy. The 1-series PGs in particular have desirable actions and the inventor has seen that if the steroid action in blocking formation of all PGs and other products can be balanced by selective restoration of 1-series PG formation, a desirable therapeutic result will occur, especially in long term use. This situation is achieved by combining a glucocorticoid ointment or other preparation with a precursor of 1-series PGs such as linoleic acid, GLA or DGLA. These fatty acids lead to an increase in the 1-series PG precursor, DGLA, in a form which can be converted to PGE1 even in the presence of glucocorticoids.

Of the three fatty acids, GLA is the most desirable because unlike DGLA it has other effects on the skin which do not seem to depend on its conversion to PGs. GLA and linoleic acid, but not DGLA, can for example, restore normal skin permeability to water in animals deficient in essential fatty acids. Of the two, GLA is preferable to linoleic acid because of the many factors known to inhibit the $\Delta^6$-desaturase. Little of the GLA is converted through to arachidonic acid because the level of $\Delta^5$-desaturase activity is low in humans and also because of a further effect of glucocorticoids which is believed important, namely that they have been reported actually to inhibit the $\Delta^5$-desaturase (De Gomez Dumm et al, J. Lipid. Res. 20: 834-9, 1979). They would thus increase the ratio of DGLA and its products to arachidonic acid and its products. Since the products derived from arachidonic acid appear to be considerably more pro-inflammatory than those formed from DGLA, such enzyme inhibition contributes to the anti-inflammatory effect.

OTHER FATTY ACIDS

Emphasis above is on the relation of EFAs to prostaglandins. However, as noted, both the EFAs themselves and their biological cyclo-oxygenase and lipoxygenase products have independent functions in the skin. The effect of EFAs as such is well illustrated by the unusual 18:3 fatty acid, columbinic acid (n-6,9,13 trans) which cannot be converted to prostaglandins (Houtsmuller, Progr. Lipid. Res. 20: 889-96, 1982). This fatty acid is able to correct most of the skin consequences of EFA deficiency, illustrating that the fatty acids themselves have key roles in the skin. Columbinic acid is found in abundance in the seeds of Aquilegia vulgaris.

Another group of fatty acids whose function in the skin is uncertain but which are believed to have a role since they are present in significant quantities is the n-3 series of EFAs derived from $\alpha$-linolenic acid.

Glucocorticoid inhibition of phospholipase enzymes is to be expected to limit the availability of the n-3 acids to the skin also. They are set out with the relation of their conversion pathway to that of the n-6 EFAs in the following table:

| n-6 | n-3 |
|---|---|
| 18:2 $\Delta^{9,12}$ (linoleic acid) | 18:3 $\Delta^{9,12,15}$ ($\alpha$-linolenic acid) |
| $\downarrow$ $\Delta^6$ desaturase | $\downarrow$ |
| 18:3 $\Delta^{6,9,12}$ ($\gamma$-linolenic acid) | 18:4 $\Delta^{6,9,12,15}$ |
| $\downarrow$ elongation | $\downarrow$ |
| 20:3 $\Delta^{8,11,14}$ (dihomo-$\gamma$-linolenic acid) | 20:4 $\Delta^{8,11,14,17}$ |
| $\downarrow$ $\Delta^5$ desaturase | $\downarrow$ |
| 20:4 $\Delta^{5,8,11,14}$ (arachidonic acid) | 20:5 $\Delta^{5,8,11,14,17}$ |
| $\downarrow$ elongation | $\downarrow$ |
| 22:4 $\Delta^{7,10,13,16}$ (adrenic acid) | 22:5 $\Delta^{7,10,13,16,19}$ |
| $\downarrow$ $\Delta^4$ desaturase | $\downarrow$ |
| 22:5 $\Delta^{4,7,10,13,16}$ | 22:6 $\Delta^{4,7,10,13,16,19}$ |

The pathways are not normally reversible nor, in man, are n-3 and n-6 series acids interconvertible.

The acids, which naturally are of the all-cis configuration, are systematically named as derivatives of the corresponding octadecanoic, eicosanoic or docosanoic acids e.g. $\Delta^{9,12}$octadecadienoic acid or $\Delta^{4,7,10,13,16,19}$docosahexaenoic acid, but numerical designation such as, correspondingly, 18:2 n-6 or 22:6 n-3 is convenient. Initials, for example, DHA for 22:6 n-3 (docosahexaenoic acid), are also used but do not serve when n-3 and n-6 acids of the same chain length and degree of unsaturation exist. Trivial names in more or less common use in the n-6 series are as shown. Of the n-3 series only 18:3 n-3 has a commonly used trivial name, $\alpha$-linolenic acid. It was characterised earlier than $\gamma$-linolenic acid and reference in the literature simply to linolenic acid, especially in the earlier literature is to the $\alpha$-acid.

In further regard to the significance of the n-3 series acids it may be noted that as between the n-6 and n-3 acids the elongation reactions (e.g. GLA to DGLA) are highly efficient and there is very little competition either way. In contrast, the two series of fatty acids are in competition in the desaturation processes. The n-3 fatty acids interfere with both $\Delta^6$ and $\Delta^5$ desaturation in the n-6 series. This competition seems to occur even when the n-3 fatty acid is not actually a substrate for the enzyme concerned. For example, 20:5 n-3 competitively inhibits the $\Delta^6$ desaturation forming GLA from linoleic acid and overall the presence of n-3 fatty acids in a combination leads to some inhibition of the conversion of DGLA to arachidonic acid by the $\Delta^5$ desaturase. As a result of the presence of n-3 EFAs, the efficiency of either GLA or DGLA in increasing the ratio of DGLA products (1-series PGs) to arachidonic acid products (2-series PGs) will therefore be increased so that compositions in which n-3 acids accompany GLA or DGLA are of particular value.

PRIOR ART

The above would indicate a broad statement of the present invention but in May, 1959 Chemisch -Pharmazeutische Fabrik GmbH published a leaflet which, in English translation, reads:- "Linola-H-Fett. New 0.2% Prednisolone in a water/oil emulsion with linoleic-linolenic acid (vitamin F), 65% fat content, and a mixture of provitamin A, vitamin $D_2$ and Vitamin E.
Dermatoses with dry skin (neuro-dermatitis); juvenile and infantile eczema; crusta lactea; eczema of old age; and any irritable skin. 10g tube. DM 4.90".

THE INVENTION

In the light of the above, the invention is claimed in terms of:-
(a) The use, for the manufacture of a medicament for combating the reduction of 1-series prostaglandin production and of free gamma-linolenic acid level in the skin in the course of anti-inflammatory glucocorticoid treatment of the skin, of the glucocorticoid in conjunction with one or more essential fatty acids, wherein the or each said essential fatty acid is of the n-6 series, gamma-linolenic acid and its metabolites, and/or the n-3 series, delta-6,9,12,15-octadecatetraneoic acid and its metabolites, and wherein said medicament is a topical preparation containing by weight, 0.01 to 30% fatty acid and 0.01 to 10% glucocorticoid in a topical application base.
(b) The use, for the manufacture of a medicament for combating the reduction of 1-series prostaglandin production and of free gamma-linolenic acid level in the skin in the course of anti-inflammatory glucocorticoid treatment of the skin with a topical preparation comprising 0.01 to 10% glucocorticoid by weight, of an oral or internally absorbable preparation comprising one or more essential fatty acids of the n-6 series, gamma-linolenic acid and its metabolites, and/or the n-3 series delta-6,9,12,15-octadecatetraneoic acid and its metabolites, the amount of said essential fatty acid being from 0.1 to 10 g or sub-multiple suited to daily administration of that amount.

In particular the fatty acids may be selected from gamma-linolenic acid, dihomo-gamma linolenic acid, the 22:4 and 22:5 n-6 essential fatty acids, the 18:4, 20:4, 20:5, 22:5 and 22:6 n-3 essential fatty acids, and columbinic acid.

Preferably, for the specific effect on PG balance, the acids are the earlier n-6 EFAs linoleic acid, GLA and DGLA of which for the reasons explained GLA and DGLA are preferred and GLA is best.

Anti-inflammatory glucocorticoids are a well recognised class of natural or synthetic steroids having biological effects similar to naturally occurring hydrocortisone (cortisol) and are fully described in, for example, Martindale's Extra Pharmacopeia, 27th edition, 1977, pages 389 to 440, or in Goodman and Gilman Pharmacological Basis of Therapeutics, 6th edition, pages 1470 to 1492, 1980 to which reference may be made. Examples include hydrocortisone itself, cortisone, betametasone, dexamethasone, fluprednisolone, methylprednisolone, paramethasone, prednisone, prednisolone, triamcinolone, beclomethasone, clobetasol, cloprednol, cortivazol, deoxycortone, desonide, desoxymethasone, diflucortolone, fluclorolone, fludrocortisone, flumethasone, flunisolide, flucinolone, fluocinonide, fluocortolone, fluoromethalone, fluperolone, fluprednidene, fluradrenolone, formocortal, halcinonide, hydrocortamate, medrysone, methylprednisone, paramethasone, prednisolamate and prednylidene.

The anti-inflammatory glucocorticoids treatment may for example be for contact dermatitis, atopic dermatitis, psoriasis or seborrhoeic dermatitis.

## FORMS OF ESSENTIAL FATTY ACID

The acids may be used as such or in any physiologically compatible form equivalent to them, for example, those referred to for gamma-linolenic acid below and general reference to the acids herein, including in the claims, is to be taken as including such derivatives. Equivalence is demonstrated by entering into the pathways quoted herein as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al page 23, "Analysis of Lipids and Lipoproteins" Ed. Perkins, American Oil Chemists Society, Champaign, Illinois, U.S.A.

In outline the method is suitably that plasma samples (1 ml) are extracted with chloroform:methanol (2:1). The extract is filtered through sodium sulphate, evaporated to dryness and taken up in 0.5 ml chloroform:methanol. The lipid fractions are separated by thin layer chromatography on silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot column packed with 10% silar on chromosorb WAW 106/230. The carrier gas is helium (30ml/min). Oven temperature is programmed to rise from 165°C to 190°C at 2°C/min. Detector temperature is 220°C and injector temperature 200°C. Retention times and peak areas are automatically computed by Hewlett-Packard Level 4 integrator. Peaks are identified by comparison with standard fatty acid methyl esters.

## PACKS

If it is not desired to have compositions comprising different active materials together, packs may be prepared comprising the materials presented for separate, or part joint and part separate use in the appropriate relative amounts, and such packs are within the purview of this invention. In particular the glucocortocoid may be for application to the skin but the EFA as an oral or other preparation to be used at the same time. Such preparation may be in a suitable pharmaceutical vehicle, as discussed in detail for example in Williams British Patent Specification No. 1 082 624, to which reference may be made, and in any case very well known generally for any particular kind of preparation. Thus, for example, tablets, capsules, ingestible liquid or powder preparations can be prepared as required. Injectable solutions of hydrolysed Oenothera oil may be prepared using albumin to solubilise the free acid. The amounts of EFA are such as to give daily dosages of 0.1 mg to 10 g preferably 30 mg to 1 g.

## VETERINARY APPLICATIONS

It will be understood that where a disorder of a kind calling for treatment in animals arises, the invention whilst described primarily in terms of human medicine is equally applicable in the veterinary field.

## FORMS AND SOURCES OF $\gamma$-LINOLENIC AND DIHOMO-$\gamma$-LINOLENIC ACIDS

Convenient physiologically functional derivatives of $\gamma$-linolenic acid and dihomo-$\gamma$-linolenic acid for use according to the invention include salts, amides, esters including glycerides and alkyl (e.g. $C_1$ to $C_4$) esters, and phospholipids.

If desired, pharmaceutical compositions may be produced for use in the invention by associating the natural or synthetic acids, as such or as derivatives, with an acceptable pharmaceutical vehicle. It is, however, at present convenient to incorporate at least the $\gamma$-linolenic acid into compositions in the form of an available oil having a high $\gamma$-linolenic acid content, hence references to "oil" herein.

At the present time known natural sources of oils having a high $\gamma$-linolenic acid content are few (there are no known natural sources of significant amounts of dihomo-$\gamma$-linolenic acid). One source of oils currently available is the seed of Evening Primrose species such as Oenothera biennis L. and Oenothera lamarc-

5

kiana, the oil extract therefrom containing γ-linolenic acid (about 8%) and linoleic acid (about 72%) in the form of of their glycerides together with other glycerides (percentages based on total fatty acids). Other sources of γ-linolenic acid are Borage species such as Borago officinalis which, though current yield per acre is low, provides a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic and linoleic as the main fatty acid components, the γ-linolenic acid content being if desired a major proportion. Seed oil extracts appear to have a stabilising effect upon dihomo-γ-linolenic acid if present.

The Oenothera oil is extracted from the seeds by one of the conventional methods of extraction such as cold pressure, screw pressure after partially cooking the seed, or solvent extraction.

Fractionation of a typical sample of this oil in the form of methyl esters shows the relative proportions:

| Palmitate | 6.15 |
| Stearate | 1.6 |
| Oleate | 10.15 |
| Linoleate | 72.6 |
| γ-Linolenate | 8.9 |

As preservative, α-tocopherol may be added to the oil in a concentration of 0.1%.

EXAMPLES

The following Examples are of ointments for use against psoriasis and against contact, atopic or seborrhoeic dermatitis applied one or more times daily.

In each of these Examples, the preparation is made up to 100% with a cream, ointment, lotion or other base suitable for topical application as standard in the art.

| EXAMPLE 1 | |
| --- | --- |
| Hydrocortisone | 1% |
| GLA | 1% |

| EXAMPLE 2 | |
| --- | --- |
| Hydrocortisone | 2% |
| EPA | 1% |

| EXAMPLE 3 | |
| --- | --- |
| Hydrocortisone | 1% |
| Columbinic acid | 2% |

| EXAMPLE 4 | |
| --- | --- |
| Hydrocortisone | 2% |
| GLA | 1% |
| EPA | 0.1% |
| Columbinic acid | 1% |

EP 0 173 478 B1

| EXAMPLE 5 | |
|---|---|
| Hydrocortisone | 1% |
| GLA | 2% |
| EPA | 0.5% |

| EXAMPLE 6 | |
|---|---|
| Hydrocortisone | 2% |
| GLA | 2% |
| Columbinic acid | 1% |

In the above formulations, hydrocortisone may be replaced by equivalent steroids, e.g. prednisolone 0.5%, triamcinolone 0.1%, betamethasone 0.1%.

A pack may comprise a topical ointment with such amounts of steroid, together with capsules of the Oenothera oil above containing 0.5 g oil extract = ca. 0.045 g gamma-linolenic acid to be taken in the requisite amounts.

## Claims

1. The use, for the manufacture of a medicament for combating the reduction of 1-series prostaglandin production and of free gamma-linolenic acid level in the skin in the course of anti-inflammatory glucocorticoid treatment of the skin, of the glucocorticoid in conjunction with one or more essential fatty acids, wherein the or each said essential fatty acid is of the n-6 series, gamma-linolenic acid and its metabolites, and/or the n-3 series, delta-6,9,12,15-octadecatetraneoic acid and its metabolites, and wherein said medicament is a topical preparation containing by weight, 0.01 to 30% fatty acid and 0.01 to 10% glucocorticoid in a topical application base.

2. A method according to claim 1, wherein the or each fatty acid is selected from gamma-linolenic acid, dihomo-gamma linolenic acid, the 22:4 and 22:5 n-6 essential fatty acids, the 18:4, 20:4, 20:5, 22:5 and 22:6 n-3 essential fatty acids, and columbinic acid.

3. A method according to claim 1 or 2, wherein the glucocorticoid is hydrocortisone, cortisone, betametasone, dexamethasone, fluprednisolone, methylprednisolone, paramethasone, prednisone, prednisolone, triamcinolone, beclomethasone, clobetasol, cloprednol, cortivazol, deoxycortone, desonide, desoxymethasone, diflucortolone, fluclorolone, fludrocortisone, flumethasone, flunisolide, flucinolone, fluocinonide, fluocortolone, fluoromethalone, fluperolone, fluprednidene, fluradrenolone, formocortal, halcinonide, hydrocortamate, medrysone, methylprednisone, paramethasone, prednisolamate and prednylidene.

4. The use, for the manufacture of a medicament for combating the reduction of 1-series prostaglandin production and of free gamma-linolenic acid level in the skin in the course of anti-inflammatory glucocorticoid treatment of the skin with a topical preparation comprising 0.01 to 10% glucocorticoid by weight, of an oral or internally absorbable preparation comprising one or more essential fatty acids of the n-6 series, gamma-linolenic acid and its metabolites, and/or the n-3 series delta-6,9,12,15-octadecatetraneoic acid and its metabolites, the amount of said essential fatty acid being from 0.1 to 10 g or sub-multiple suited to daily administration of that amount.

## Patentansprüche

1. Die Verwendung zur Herstellung eines Medikamentes zur Bekämpfung der Reduzierung der Produktion von Prostaglandinen der Reihe 1 und des freien Gammalinolensäuregehalts in der Haut im Laufe der entzündungshemmenden Behandlung der Haut mit Glukokortikoiden, des Glukokortikoids zusammen mit einer oder mehreren essentiellen Fettsäuren, wobei die oder jede einzelne der genannten essentiellen Fettsäuren eine Säure der Reihe n-6, Gammalinolensäure und ihre Metaboliten, und/oder der Reihe n-3, Delta-6,9,12,15-Oktadekatetraensäure und ihre Metaboliten ist und wobei das besagte Medikament

7

eine Präparation zur äußeren Anwendung ist, die 0,01 bis 30 Gewichtsprozent Fettsäure und 0,01 bis 10 Gewichtsprozent Glukokortikoid auf der Basis einer äußeren Anwendung enthält.

2. Ein Verfahren gemäß Anspruch 1, wobei die oder jede Fettsäuren ausgewählt werden aus Gammalinolensäure, Dihomogammalinolensäure, den essentiellen Fettsäuren 22:4 und 22:5 n-6, den essentiellen Fettsäuren 18:4, 20:4, 20:5, 22:5 und 22:6 n-3 und der Niobsäure.

3. Ein Verfahren gemäß Anspruch 1 oder 2, wobei das Glukokortikoid Hydrocortison, Cortison, Betamethason, Dexamethason, Fluprednisolon, Methylprednisolon, Paramethason, Prednison, Prednisolon, Triamcinolon, Beclometason, Clobetasol, Cloprednol, Cortivazol, Desoxycorton, Desonid, Desoxymethason, Diflucortolon, Fluclorolon, Fludrocortison, Flumethason, Flunisolid, Flucinolon, Fluocinoid, Fluocortolon, Fluorometholon, Fluperolon, Flupredniden, Fluradrenolon, Formocortol, Halcinonid, Hydrocortamat, Medryson, Methylprednison, Paramethason, Prednisolamat und Prednyliden ist.

4. Die Verwendung zur Herstellung eines Medikamentes zur Bekämpfung der Reduzierung der Produktion von Prostaglandinen der Reihe 1 und des freien Gammalinolensäuregehalts in der Haut im Laufe der entzündungshemmenden Behandlung der Haut mit Glukokortikoiden mit einer Präparation zur äußeren Anwendung, die zwischen 0,01 und 10 Gewichtsprozent Glukokortikoid enthält, einer oral oder innerlich absorptionsfähigen Präparation, die eine oder mehrere essentielle Fettsäuren der Reihe n-6, Gammalinolensäure und ihre Metaboliten, und/oder der Reihe n-3, Delta-6,9,12,15-Oktadekatetraensäure und ihre Metaboliten ist und die Menge der besagten essentiellen Fettsäure zwischen 0,1 und 10 g oder einen ohne Rest teilbarer Teil von diesen beträgt, der für die tägliche Verabreichung dieser Menge geeignet ist.

**Revendications**

1. Utilisation pour préparer un médicament destiné à lutter contre la réduction de la production des prostaglandines de la série 1 et du taux cutané de l'acide gamma-linolénique libre au cours d'un traitement de la peau par des glucocorticoïdes anti-inflammatoires, du glucocorticoïde conjointement à un ou plusieurs acides gras essentiels, où l'acide gras essentiel, ou chacun d'entre eux, est l'acide gamma-linolénique de la série n-6, ainsi que ses métabolites, et/ou l'acide delta-6,9,12,15-octadécatétraénoïque de la série n-3 et ses métabolites, le médicament étant une préparation à usage local contenant de 0,01 à 30 % en poids d'un acide gras et de 0,01 à 10 % en poids d'un glucocorticoïde dans une base pour application locale.

2. Procédé selon la revendication 1, dans lequel l'acide gras, ou chacun d'eux, est choisi parmi l'acide gamma-linolénique, l'acide dihomo-gamma-linolénique, les acides gras essentiels en n-6 22:4 et 22:5, les acides gras essentiels en n-3 18:4, 20:4, 20:5, 22:5, et 22:6, et l'acide colombinique.

3. Procédé selon la revendication 1 ou 2, dans lequel le glucocorticoïde est l'hydrocortisone, la cortisone, la bétaméthasone, la dexaméthasone, la fluprednisolone, la méthylprednisolone, la paraméthasone, la prednisone, la prednisolone, la triamcinolone, la béclométhasone, le clobétasol, le cloprednol, le cortivasol, la déoxycortone, le désonide, la désoxyméthasone, le diflucortolone, la fluclorolone, la fludrocortisone, la fluméthasone, le flunisolide, la flucinolone, le fluocinonide, la fluocortolone, la fluorométhalone, la flupérolone, le fluprednidène, la fluradrénolone, le formocortal, l'halcinonide, l'hydrocortamate, la médrysone, la méthylprednisone, la paraméthasone, le prednisolamate et le prednylidène.

4. Utilisation pour préparer un médicament destiné à lutter contre la réduction de la production des prostaglandines de la série 1 et du taux cutané de l'acide gamma-linolénique libre au cours du traitement de la peau par des glucocorticoïdes anti-inflammatoires, avec une préparation locale comprenant de 0,01 à 10 % en poids d'un glucocorticoïde, d'une préparation orale ou pouvant être absorbée par voie interne comprenant un ou plusieurs acides gras essentiels de la série n-6, de l'acide gamma-linolénique et ses métabolites, et/ou l'acide delta-6,9,12,15-octadécatétraénoïque de la série n-3 et ses métabolites, la quantité de l'acide gras essentiel étant de 0,1 à 10 g ou un sous-multiple de cette quantité, convenant à une administration journalière.